(19) Europäisches Patentamt
European Patent Office
Office européen des brevets

(11) Veröffentlichungsnummer: **0 502 594 A1**

(12) # EUROPÄISCHE PATENTANMELDUNG

(21) Anmeldenummer: **92250051.7**

(22) Anmeldetag: **05.03.92**

(51) Int. Cl.5: **C07C 215/50**, C07C 251/38, C07C 309/46, C07C 323/32, C07C 331/28, C07D 207/40, C07D 213/70, A61K 49/02, A61K 43/00

(30) Priorität: **07.03.91 DE 4107570**

(43) Veröffentlichungstag der Anmeldung: **09.09.92 Patentblatt 92/37**

(84) Benannte Vertragsstaaten: **AT BE CH DE DK ES FR GB GR IT LI LU NL PT SE**

(71) Anmelder: **INSTITUT FÜR DIAGNOSTIKFORSCHUNG GmbH AN DER FREIEN UNIVERSITÄT BERLIN**
**Klinikum Rudolf Virchow, Spandauer Damm 130**
**W-1000 Berlin 19(DE)**

(72) Erfinder: **Neumeier, Reinhard, Dr.**
**Gabrielenstrasse 63**
**W-1000 Berlin 27(DE)**
Erfinder: **Kramp, Wolfgang, Dr.**
**Damwildsteig 41a**
**W-1000 Berlin 27(DE)**
Erfinder: **Mäcke, Helmut, Dr.**
**Bergfriedweg 7**
**W-7850 Lörrach(DE)**
Erfinder: **Rohlfs, Gerhard, Dr.**
**Sandhausener Strasse 24**
**W-1000 Berlin 27(DE)**

(54) **Chelate, deren Metallkomplexe sowie ihre Verwendung in Diagnostik und Therapie.**

(57) Die Erfindung betrifft Verbindungen der allgemeinen Formel I

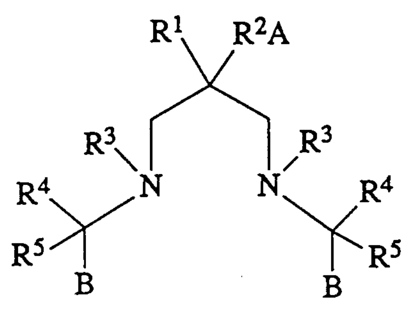

(I)

worin A eine funktionelle Gruppe beinhaltet, die für die Kopplung an sich selektiv anreichernde Verbindungen geeignet ist und $R^1$, $R^2$, $R^3$, $R^4$ und $R^5$ unterschiedliche Bedeutung haben können. B ist eine Gruppe, die für die koordinative Bindung von Metallionen geeignet ist. Die neuen Verbindungen dienen zur Komplexierung von radioaktiven Metallionen, insbesondere Rhenium- und Technetium-Isotopen, und werden in der medizinischen Diagnostik und Therapie eingesetzt.

EP 0 502 594 A1

Seit längerer Zeit werden radioaktive Metallionen, meist gebunden an einen Komplexbildner, für die in vivo-Diagnostik verwendet. Hiervon ist Technetium-99m (Tc-99m) aufgrund seiner für diese Zwecke nahezu idealen physikalischen Eigenschaften- gute Absorption der Strahlung in entsprechenden Detektionsgeräten (Gammakamera, SPECT-Geräte) gegenüber einer geringen Absorption im menschlichen Organismus und leichte Verfügbarkeit über einen Molybdän/Technetium-Generator - das am häufigsten in der klinischen Nuklearmedizin verwendete Radio -nuklid. Seine kurze Halbwertszeit von 6,02 h garantiert eine nur geringe Belastung des Patienten mit Gammastrahlung, zumal auch das Tochternuklid Technetium-99 nur eine vernachlässigbare Reststrahlung besitzt. Ein Nachteil des Technetiums ist allerdings seine komplizierte und noch nicht vollständig bekannte Komplex-Chemie. Technetium kann in einer Reihe von Oxidationsstufen (+7 bis -1) vorliegen, die die pharmakologischen Eigenschaften durch Veränderung der Ladung eines Komplexes stark verändern können. Es ist deswegen nötig, Komplexe zu synthetisieren, die das Technetium in einer definierten Oxidationstufe binden und Redox-Reaktionen, die zu einer Redistribution des Pharmakons führen könnten, verhindern. Eine Reihe solcher Tc-99m-Komplexbildner sind bereits bekannt und werden klinisch angewendet. Bei neutralen Komplexen handelt es sich vielfach um Systeme, in denen das Tc-99m zwischen 2-4 Stickstoffatomen und 0-2 Schwefelatomen gebunden wird ($N_2S_2$,- $N_3S$- und Propylenaminoximkomplexe). Vielfach ist jedoch die ungenügende Stabilität dieser Tc-99m-Komplexe ein wesentlicher Nachteil (Hung, J.C. et al.; J. Nucl. Med. 29: 1568, 1988). In der klinischen Anwendung muß deswegen z.B. HMPAO (Hexamethyl-propylenaminoxim) kurz nach seiner Markierung mit Pertechnetat appliziert werden, damit der Anteil an Zerfallsprodukten, die die diagnostische Aussagekraft vermindern, nicht zu hoch wird. Eine Kopplung dieser Chelate bzw. Chelatbildner an andere, sich selektiv in Krankheitsherden anreichernde Substanzen ist nicht möglich. Daher verteilen sich die meisten der erwähnten Komplexe entsprechend der Durchblutung und/oder metabolischen Aktivität eines Organs (z.B. Europ. Patent Appl. 0 194 843), so daß z.B. nekrotische oder ischämische Regionen nach Infarkt oder Schlaganfall in einem Szintigramm dargestellt werden können.

Für eine erfolgreiche Diagnostik von Tumoren, neurologischen Erkrankungen oder Herz-Kreislauferkrankungen sind jedoch Substanzen vielversprechender, die molekulare Veränderungen der erkrankten Gewebe darstellen können, indem sie spezifisch an diese erkrankten Gewebe binden bzw. in deren Stoffwechsel eingeschleust werden. Die Erkenntnisse der biologischen und biochemischen Grundlagenforschung erlauben die Auswahl einer Reihe von Substanzen, die sich in Krankheitsherden selektiv anreichern: Diverse Tumoren entwickeln erhöhte oder erniedrigte Oberflächenkonzentrationen an Rezeptoren z.B. für Wachstumsfaktoren oder Steroidhormone (Sledge, G.W.; Adv. Cancer Res. 38: 61-75 [1983]). Auch bei neurologischen Erkrankungen kommt es zu einer Veränderung der Konzentration von Rezeptoren für Neurotransmitter in bestimmten Bereichen des Hirns (Frost, J.J.; Trends Pharmacol. Sci. 7: 490-496 [1987]). Weiterhin zeigen erkrankte, geschädigte oder zu Tumorzellen transformierte Zellen oft starke Veränderungen ihres Metabolismus und einen Sauerstoffmangel im Innern des Tumors. Die Ausnutzung solcher physiologischer Besonderheiten kann der in vivo-Diagnostik dienen, indem z.B. Hormone, Neurotransmitter oder bestimmte Stoffwechselprodukte wie Fettsäuren, Saccharide, Peptide oder Aminosäuren an Chelatbildner für Tc-99m gekoppelt werden. Auch Substanzen wie Misonidazol (ein Radiosensitizer) bzw. andere in Abwesenheit von Sauerstoff sich zu Radikalen umsetzende Verbindungen können zur spezifischen Anreicherung von radioaktiven Isotopen und somit bildlichen Darstellung von Tumoren oder ischämischen Regionen herangezogen werden. Schließlich ist auch die Kopplung an monoklonale Antikörper möglich, die aufgrund ihrer hohen Spezifität zu einem vielversprechenden Instrument in der Tumordiagnostik geworden sind.

Für die Herstellung von Diagnostika nach dem beschriebenen Prinzip ist es notwendig, daß Chelatbildner für radioaktive Metallionen, insbesondere Tc-99m, an in erkrankten Geweben sich selektiv anreichernde Substanzen gekoppelt werden können.

Da die Isotope des Rheniums (Re-188 und Re-186) ähnliche chemische Eigenschaften wie Tc-99m besitzen, können die Chelatbildner auch zur Komplexierung dieser Isotope verwendet werden. Die genannten Re-Isotope sind $\beta$-Strahler. Somit sind die sich selektiv anreichernden Substanzen mit Rhenium statt mit Technetium komplexiert auch in der Tumortherapie einsetzbar.

Die bisher bekannten Ansätze zur Kopplung von Chelatbildnern an sich selektiv anreichernde Substanzen können vielfach als nicht zufriedenstellend betrachtet werden. Werden die funktionellen Gruppen des Komplexbildners zur Bindung des Chelatbildners an ein solches Molekül benutzt, so kommt es häufig zu einer Abschwächung der Komplexstabilität, d.h. ein diagnostisch nicht tolerierbarer Anteil des Isotops wird aus dem Konjugat freigesetzt. (Brechbiel, M. W. et.al., Inorg. Chem. 25:2772 [1986]). Es ist deswegen notwendig bifunktionelle Komplexbildner herzustellen, d.h. Komplexbildner, die sowohl funktionelle Gruppen zur koordinativen Bindung des gewünschten Metallions als auch eine (andere) funktionelle Gruppe zur Bindung des sich selektiv anreichernden Moleküls tragen. Solche bifunktionellen Liganden ermöglichen eine spezifische, chemisch definierte Bindung von Technetium an verschiedenste biologische Materialien, auch

dann, wenn ein sogenanntes Prelabeling durchgeführt wird. Da nach dieser Methode zuerst die Markierung mit Tc-99m und die Isolierung der Komplexe durchgeführt und erst in einem zweiten Schritt dieser Komplex mit einem sich selektiv anreicherndem Molekül verknüpft wird, erhält man die markierten Verbindungen mit einem hohen Reinheitsgrad.

Es wurden einige Chelatbildner gekoppelt an monoklonale Antikörper (z.B. Europ. Patent App. 0 247 866 und 0 188 256) oder Fettsäuren (Europ. Patent Appl. 0 200 492) beschrieben. Als Chelatbildner werden jedoch die bereits erwähnten $N_2S_2$-Systeme verwendet, die aufgrund ihrer geringen Stabilität wenig geeignet sind. Die etwas stabileren $N_3S$-Chelate zeigten gekoppelt an monoklonale Antikörper keinen so starken Verlust des Tc-99m aus den Konjugaten (J. Lister-James; J. Nucl. Med. 30: 793 und Europ. Patent Appl. 0 284 071).

Da sowohl die sich selektiv anreichernden Substanzen in ihren Eigenschaften, sowie auch die Mechanismen, nach denen sie angereichert werden, sehr unterschiedlich sind, ist es weiterhin notwendig, den kopplungsfähigen Chelatbildner variieren und den physiologischen Anforderungen des Kopplungspartners hinsichtlich Lipo- und Hydrophilie, Membranpermeabilität bzw. -impermeabilität etc. anpassen zu können.

Es besteht aus diesen Gründen ein dringlicher Bedarf an stabilen Komplexverbindungen, die gekoppelt oder fähig zur Kopplung an unterschiedliche sich selektiv anreichernde Verbindungen sind.

Aufgabe der Erfindung ist es somit, stabile Chelatbildner, die eine funktionelle Gruppe zur Kopplung an eine sich selektiv anreichernde Verbindung oder eine mit Hilfe dieser funktionellen Gruppe gekoppelte sich selektiv anreichernde Verbindung enthalten, zur Verfügung zu stellen.

Erfindungsgemäß wird diese Aufgabe durch die Verbindungen der allgemeinen Formel I gelöst,

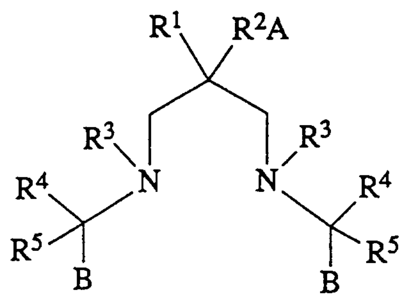

(I)

worin $R^1$ für Wasserstoff oder einen gegebenenfalls mit ein oder zwei Hydroxylgruppen substituierten $C_{1-6}$-Alkylrest,

$R^2$ für einen gegebenenfalls mit einer Hydroxylgruppe substituierten $C_{1-6}$-Alkylenrest,

$R^3$ für ein Wasserstoffatom, einen $C_{1-6}$-Alkyl-, einen Carboxymethyl- oder einen ($C_{1-6}$ Alkoxycarbonyl)-methylrest,

$R^4$ für ein Wasserstoffatom, einen gegebenenfalls mit einer Hydroxylgruppe substituierten $C_{1-6}$-Alkylrest bzw. für die unter $R^x$ angegebene Bedeutung steht,

$R^5$ für ein Wasserstoffatom oder einen gegebenenfalls mit einer Hydroxylgruppe substituierten $C_{1-6}$-Alkylrest steht,

B für einen Pyrrolyl-, einen substituierten Phenylrest der Formel II oder einen substituierten Pyridinrest der Formel III

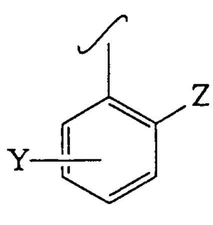

(II)

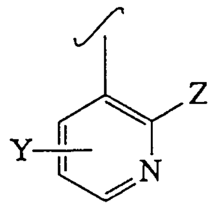

(III)

worin Z eine Hydroxyl-, Amino- oder eine Mercaptogruppe und Y ein Wasserstoffatom, einen Carboxy- oder einen Sulfonylrest bedeuten

oder für einen Nitrosomethylrest der Formel IV

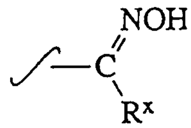

(IV)

steht,

worin $R^x$ einen $C_{1-6}$-Alkylrest, der gegebenenfalls zusammen mit $R^4$ über eine Trimethylen-oder Tetramethylengruppe zu einem 5-bzw. 6-Ring cyclisiert ist, bedeutet
und A für einen Amino-, einen Mercapto-, einen Carboxy-, einen $C_{2-6}$-Alkinyl- oder -Alkenyl-, einen Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht,

wobei die Phenylgruppe gegebenfalls zusätzlich durch einen Carboxy-, einen Chlorsulfonyl- oder einen Sulfonsäurerest substituiert sein kann

oder eine - mit Hilfe der genannten funktionellen Gruppe gegebenenfalls über einen bifunktionellen Linkerrest L gebundene- sich selektiv in Läsionen oder bestimmten Geweben anreichernde Verbindung T enthält

wobei T für monoklonale Antikörper oder deren Fragmente, Hormone, Enzyme, Liganden für Zellmembranrezeptoren, Neurotransmitter, Lipide, Steroide, Saccharide, Aminosäuren und Oligopeptide, Biotin, sowie Radiosensitizer, wie z.B. Misonidazol steht,

wobei im Falle von

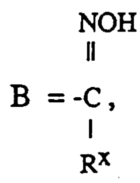

Pyrrolyl- oder Y = H, $R^2$ für eine hydroxilierte $C_{1-6}$-

Alkylenkette oder A für einen substituierten Aminophenyl- oder Isothiocyanatophenylrest stehen sollen sowie deren Komplexe mit- zur Diagnostik und Tumortherapie geeigneten - radioaktiven Metallionen, sowie deren Salze mit anorganischen und organischen Säuren und Basen.

Erfindungsgemäß bevorzugt sind diejenigen Verbindungen gemäß Anspruch 1, in denen $R^4$ und $R^5$ Wasserstoffatome oder Methylreste bedeuten sowie diejenigen Verbindungen gemäß Anspruch 1, in denen A für einen Amino-, einen Mercapto-, einen Carboxy-, einen $C_{2-6}$-Alkinyl- oder -Alkenyl- einen Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht, wobei die Phenylgruppe durch einen weiteren Carboxyl- oder Sulfonsäurerest substituiert sein kann und die gegebenenfalls in A enthaltene sich selektiv anreichernde Verbindung T für monoklonale Antikörper, deren Fragmente, Biotin oder Misonidazol steht.

Überraschenderweise zeigten viele der synthetisierten und mit Tc-99m markierten Chelate eine höhere Stabilität als die vergleichbaren $N_2S_2$-, $N_3S$- und Propylenaminoxim-Chelate. So konnten z.B. bei einer erfindungsgemäßen Substanz (Beispiel 2), die über einen Aminophenylrest an Biotin gekoppelt wurde, keine Zersetzung-Produkte nach 5 Std. beobachtet werden, während dies bei dem vergleichbaren, literaturbekannten HMPAO der Fall war (Hung, J.C. et al.; J. Nucl. Med 29: 1568, 1988). Auch durch Kompetitionsversuche konnte festgestellt werden, daß die in dieser Erfindung beschriebenen Chelate Tc-99m besser als die vergleichbaren $N_2S_2$-, $N_3S$- und Propylenaminoxim-Chelaten komplexieren. Die in der Erfindung beschriebenen Chelate sind damit eindeutig besser für diagnostische und therapeutische Zwecke geeignet als die bisher bekannten Chelate.

Die Herstellung der Verbindungen gemäß Anspruch 1 erfolgt, indem man

a) ein 1,3-Propandiamin der allgemeinen Formel V

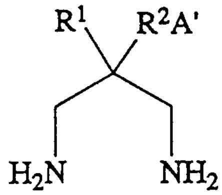

(V)

worin $R^1$ und $R^2$ die oben genannten Bedeutungen haben, und A' A oder ein in A umwandelbarer Rest bedeutet
mit einer Verbindung der allgemeinen Formel VI

B - $R^4$C = O (VI)

in der $R^4$ und B die oben genannten Bedeutungen haben,
in einem polaren Lösungsmittel, vorzugsweise Ethanol, oder unter Verwendung eines Wasserabscheiders in einem unpolaren Lösungsmittel, vorzugsweise Benzol, bei Temperaturen von 25 - 180 ° C innerhalb von 6 Stunden bis 3 Tagen umsetzt, die Iminofunktion in an sich bekannter Weise, vorzugsweise mit Natriumborhydrid in einem polaren Lösungsmittel, vorzugsweise einem Methanol/Wasser-Gemisch, bei Temperaturen von 25 - 100 ° C innerhalb von 0,5 bis 24 Stunden, vorzugsweise 2 Stunden, reduziert,
oder

b) indem man ein Propandiamin der allgemeinen Formel V

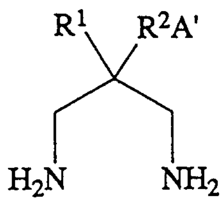

(V)

worin $R^1$, $R^2$ und A' die oben genannten Bedeutungen haben,
mit einer Verbindung der allgemeinen Formel VII

B - CO-X (VII),

in der B die oben genannte Bedeutung hat, in B enthaltene funktionelle Gruppen gegebenenfalls in geschützter Form vorliegen und X für ein Halogenatom, vorzugsweise für ein Chloratom steht,
oder

c) indem man ein Propandiamin der allgemeinen Formel V

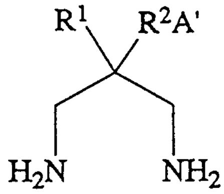

(V)

worin $R^1$, $R^2$ und A' die oben genannten Bedeutungen haben,
mit einer Verbindung der allgemeinen Formel VIII

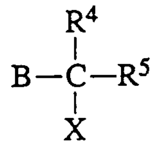

(VIII)

in der $R^4$, $R^5$, B und X die oben genannten Bedeutungen haben und in B enthaltene funktionelle Gruppen gegebenenfalls in geschützter Form vorliegen,
oder
d) substituierte Malonsäurehalogenide, vorzugsweise Malonsäurechloride der allgemeinen Formel IX,

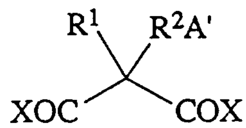

(IX)

worin $R^1$, $R^2$, A' und X die oben genannten Bedeutungen haben und in B enthaltene funktionelle Gruppen gegebenenfalls in geschützter Form vorliegen,
mit einem Amin der allgemeinen Formel X

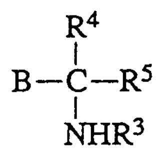

(X)

in denen $R^4$, $R^5$ und B die oben genannten Bedeutungen haben, $R^3$ für ein Wasserstoffatom oder für einen $C_{1-6}$-Alkylrest steht und in B enthaltene funktionelle Gruppen gegebenenfalls in geschützter Form vorliegen,
in einem aprotischen Lösungsmittel, vorzugsweise Dichlormethan, bei Temperaturen von 0 - 180 °C, vorzugsweise bei Raumtemperatur, innerhalb von 2 bis 24 Stunden, vorzugsweise 4 Stunden, gegebenenfalls unter Zusatz von geeigneten Basen, z.B. Triethylamin, umsetzt,
die nach Verfahren d) erhaltene Amidfunktion in an sich bekannter Weise, vorzugsweise mit Boran in THF oder mit Lithiumaluminiumhydrid in einem aprotischen Lösungsmittel, vorzugsweise Diethylether, bei Temperaturen von 25 - 150 °C innerhalb von 0,5 bis 24 Stunden, vorzugsweise 8 Stunden, zur entsprechenden Aminofunktion reduziert,
die Aminogruppen, im Falle daß $R^3$ für ein Wasserstoffatom steht, gegebenenfalls in an sich bekannter Weise mit einem Alkylierungsmittel alkyliert und vorhandene Schutzgruppen abspaltet
und in den so erhaltenen Verbindungen die Gruppe A' gegebenenfalls zu A umwandelt- gegebenenfalls nach Schutz der freien Aminogruppen und der funktionellen Gruppen Z mit Schutzionen, z.B. als Cu-Komplex - generiert und anschließend gewünschtenfalls die so erhaltenen Verbindungen über diese funktionelle Gruppe an sich selektiv anreichernde Verbindungen T koppelt und den Substituenten B gewünschtenfalls mit dem jeweils gewünschten radioaktiven Isotop komplexiert, wobei vorher im Produkt gegebenenfalls vorhandene Schutzionen nach an sich literaturbekannten Methoden entfernt werden und die Reihenfolge der Schritte Komplexierung mit radioaktiven Isotopen und Kopplung anT vertauscht werden kann.

Als Hydroxyschutzgruppen kommen z.B. die Benzyl-, 4-Methoxybenzyl-, 4-Nitrobenzyl-, Trityl-, Diphenylmethyl-, Trimethylsilyl-, Dimethyl-t-butylsilyl- und Diphenyl-t-butylsilylgruppe infrage. Im Falle von Polyolen können die Hydroxygruppen auch in Form von Ketalen mit z.B. Aceton, Acetaldehyd, Cyclohexanon oder Benzaldehyd geschützt sein. Ferner können die Hydroxygruppen auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Die Hydroxyschutzgruppen können nach den dem Fachmann bekannten Literaturmethoden, z.B. durch Hydrogenolyse, reduktive Spaltung mit Lithium / Ammoniak,

Säurebehandlung der Ether und Ketale oder Alkalibehandlung der Ester freigesetzt werden (siehe z.B. "Protective Groups in Organic Synthesis", T.W. Greene, John Wiley and Sons 1981).

Als Aminoschutzgruppen kommen z.B. Trifluoracetyl-, t-Butoxycarbonyl-, 2,2,2-Trichloroethoxycarbonyl-, Benzoxycarbonyl- und Acetylgruppen infrage. Die Aminoschutzgruppen können nach literaturbekannten Methoden, z.B. durch basische oder saure Hydrolyse, reduktive Spaltung mit Zink in Essigsäure oder Hydrogenolyse abgespalten werden.

Als Mercaptoschutzgruppen kommen $C_{1-6}$-Alkylreste oder Benzylthioether infrage. Die Abspaltung der Mercaptoschutzgruppen erfolgt wahlweise mit Alkalialkylthiolaten, Alkalialkoholaten oder Alkalimetallen, vorzugsweise mit Natriummethylthiolat in einem polaren Lösungsmittel, vorzugsweise in HMPT, DMF oder Dimethylacetamid oder durch reduktive Spaltung mit Natrium / Ammoniak.

Die funktionelle Gruppe im Rest A ist dazu geeignet, eine stabile Verbindung zu Proteinen oder anderen sich selektiv anreichernden Molekülen herzustellen. Durch die entsprechende Wahl der funktionellen Gruppe im Rest A gelingt die Kopplung unter schonenden Reaktionsbedingungen, die die biologische Funktion und/oder Selektivität nicht beeinflussen. Die Kopplung an die gewünschten Verbindungen erfolgt ebenfalls nach an sich bekannten Methoden, (z.B. Fritzberg et al.; J. Nucl. Med.: 26, 7 [1987]), beispielsweise durch Reaktion der entsprechenden funktionellen Gruppe im Rest A mit nucleophilen Gruppen des sich selektiv anreichernden Moleküls oder, wenn es sich bei der entsprechenden funktionellen Gruppe im Rest A selbst um ein Nucleophil handelt, mit aktivierten Gruppen des sich selektiv anreichernden Moleküls.

Die funktionelle Gruppe im Rest A repräsentiert jeden Substituenten, der einerseits eine Kopplung an ein sich selektiv anreicherndes Molekül unter milden Bedingungen erlaubt (z.B. durch Acylierung oder Amidierung) sowie jede aktivierte Gruppe, die mit nucleophilen Gruppen von Proteinen, Antikörpern, Hormonen oder anderen Biomolekülen reagieren kann, wie der Amino-, Phenol-, Sulfhydryl-, Formyl- oder Imidazol-Gruppe. Unter aktivierter Gruppe wird eine Funktion verstanden, die fähig ist, unter Bildung eines Konjugates mit einem nucleophilen Substituenten eines sich selektiv anreichernden Moleküls oder des Komplexliganden selbst in wässriger Lösung innerhalb einer angemessen kurzen Zeit, unter Reaktionsbedingungen, die weder Denaturierung noch Verlust der biologischen Aktivität zufolge haben, zu reagieren. Beispiele dafür sind Imidester, Alkylimidester, Amidoalkylimidester, Succinimidester, Acylsuccinimide, Phenolester, substituierte Phenolester, Tetrafluorphenolester, Anhydride, und Michael-Akzeptoren. Die funktionelle Gruppe im Rest A ist bevorzugt ein aktivierter Ester (wie Phenol- oder Imid-Ester), ein Mercaptan oder Isothiocyanat, insbesondere für die Kopplung mit nucleophilen Gruppen von Aminosäuren oder ein aliphatisches oder aromatisches primäres Amin für die Kopplung an Kohlenhydratresten von Proteinen.

Handelt es sich bei der funktionellen Gruppe im Rest A selbst um ein Nucleophil, kann sie mit aktivierten Gruppen eines sich selektiv anreichernden Moleküls reagieren, wobei auch mit sogenannten "Cross-linking-reagents" umgesetzte Gruppen des Moleküls mit eingeschlossen sind, wie homobifunktionelle Imidoester, homobifunktionelle N-Hydroxysuccinimidester (NHS) und heterobifunktionelle "Cross-Linker", die unterschiedliche funktionelle Gruppen, wie NHS-Ester, Pyridyl-Disulfide und aktivierte Halogene, wie $\alpha$-Keto-Halogenide enthalten. Solche Cross-Linker sind kommerziell erhältlich.

Als Kopplungspartner (= Verbindung T) sind u.a. verschiedene Biomoleküle vorgesehen: Liganden, die an spezifische Rezeptoren binden und so ein in ihrer Rezeptordichte verändertes Gewebe erkennen können; hierzu gehören u.a. Peptid- und Steroidhormone und Neurotransmitter. Mit Liganden für Steroidhormon-Rezeptoren wurde die Möglichkeit einer verbesserten Diagnostik von Brust- und Prostatacarcinomen aufgezeigt (S.J. Brandes & J.A. Katzenellenbogen, Nucl. Med. Biol. 15: 53, 1988). Vielfach konnten mit Positronen-emittierenden Isotopen markierte Liganden für Neurorezeptoren zur Diagnostik verschiedener Hirnerkrankungen herangezogen werden (J.J. Forst, Trends in Pharmacol. Sci. 7: 490, 1987). Weitere Biomoleküle sind in den Metabolismus der Zellen einschleusbare Metaboliten, die einen veränderten Stoffwechsel erkennbar machen; hierzu gehören beispielsweise Fettsäuren, Saccharide, Peptide und Aminosäuren. Fettsäuren gekoppelt an die unstabileren $N_2S_2$-Chelatbildner wurden in der EPA 0 200 492 beschrieben. Andere Stoffwechselprodukte wie Saccharide (Dexoxyglucose), Lactat, Pyruvat und Aminosäuren (Leucin, Methylmethionin, Glycin) wurden mit Hilfe der PET-Technik zur bildlichen Darstellung von veränderten Stoffwechselvorgängen herangezogen (R. Weinreich, Swiss Med. 8, 10, 1986). Auch nichtbiologische Substanzen wie Misonidazol und seine Derivate, die sich in Geweben bzw. Gewebeteilen mit reduzierter Sauerstoffkonzentration irreversibel an Zellbestandteile binden, können zur spezifischen Anreicherung von radioaktiven Isotopen und somit bildlichen Darstellung von Tumoren oder ischämischen Regionen herangezogen werden (M.E. Shelton, J. Nucl. Med. 30: 351, 1989). Schließlich ist auch die Kopplung der bifunktionellen Chelatbildner an monoklonale Antikörper bzw. deren Fragmente möglich. Die Biotin enthaltenden erfindungsgemäßen Verbindungen ermöglichen die Bindung der radioaktiven Konjugate an Avidin- oder Streptavidin-haltige Substanzen. Dies kann genutzt werden, um Antikörper-Streptavidin-Konjugate am Tumor anzureichern und die radioaktive Biotin-haltige Komponente erst später zu applizieren,

was zu einer verringerten Exposition des Patienten mit radioaktiver Strahlung führt (D.J. Hnatowich et al., J. Nucl. Med. 28: 1294, 1987). Durch Komplexierung der Konjugate mit Tc-99m bzw. Rhenium-Isotopen kann eine Diagnostik und Therapie von Tumoren ermöglicht werden.

Es ist unerheblich, ob eine Markierung der Chelatbildner mit Tc-99m oder einem Rhenium-Isotop vor oder nach der Kopplung an das sich selektiv anreichernde Molekül durchgeführt wird. Für eine Kopplung an das sich selektiv anreichernde Molekül nach einer Komplexierung ist jedoch Voraussetzung, daß die Umsetzung des radioaktiven Komplexes mit der sich anreichernden Verbindung schnell, unter schonenden Bedingungen und nahezu quantitativ abläuft, sowie keine anschließenden Aufreinigung erforderlich ist.

Die Herstellung der erfindungsgemäßen pharmazeutischen Mittel erfolgt in an sich bekannter Weise, in dem man die erfindungsgemäßen Komplexbildner unter Zusatz eines Reduktionsmittels, vorzugsweise Zinn-(II)-salzen wie -chlorid oder -tartrat - und gegebenenfalls unter Zugabe der in der Galenik üblichen Zusätze - in wäßrigem Medium löst und anschließend sterilfiltriert.

Geeignete Zusätze sind beispielsweise physiologisch unbedenkliche Puffer (z.B. Tromethamin), geringe Zusätze von Elektrolyten (z.B. Natriumchlorid), Stabilisatoren (z.B. Gluconat, Phosphate oder Phosphonate). Das erfindungsgemäße pharmazeutische Mittel liegt in Form einer Lösung oder in lyophilisierter Form vor und wird kurz vor der Applikation mit einer Lösung Tc-99m-Pertechnetat, eluiert aus kommerziell erhältlichen Generatoren, oder einer Perrhenatlösung versetzt

Bei der nuklearmedizinischen In-vivo-Anwendung werden die erfindungsgemäßen Mittel in Mengen von $1 \cdot 10^{-5}$ bis $5 \cdot 10^{4}$ nmol/kg Körpergewicht, vorzugsweise in Mengen zwischen $1 \cdot 10^{-3}$ und $5 \cdot 10^{2}$ nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Für therapeutische Anwendungen werden zwischen 5 und 500 mCi, vorzugsweise 10 - 350 mCi appliziert. Die Applikation wird normalerweise durch intravenöse, intraarterielle, peritoneale oder intratumorale Injektion von 0,1 bis 2 ml einer Lösung der erfindungsgemäßen Mittel erfolgen. Bevorzugt ist die intravenöse Applikation.

Die nachfolgenden Beispiele dienen der näheren Erläuterung des Erfindungsgegenstandes.

**Beispiel 1:**

2-Methyl-2-(4-nitrobenzyl)-malonsäuredimethylester[1]

Es werden 11.5 g (0.5 mol) Natrium unter einem Stickstoffstrom in einen trockenen 1000 ml fassenden Dreihalskolben mit Rückflußkühler und Trockenrohr sowie Tropftrichter mit Druckausgleich gegeben. Anschließend werden vorsichtig 350 ml Methanol zugetropft und solange gerührt, bis das Natrium vollständig gelöst ist. In die noch warme Natriummethanolat-Lösung wird sehr langsam die methanolische Lösung von 87 g (0.5 mol) Methylmalonsäurediethylester zugetropft. Nach beendeter Zugabe wird noch 30 Minuten gerührt und dann portionsweise mit Hilfe eines Pulvertrichters das 4-Nitrobenzylbromid zugegeben. Nachdem sich alles gelöst hat wird zunächst 2 Stunden unter Rückfluß erhitzt und anschließend über Nacht bei Raumtemperatur gerührt. Das Lösungsmittel wird am Rotationsverdampfer abgezogen, der Rückstand mit Wasser versetzt und mehrmals mit Ethylacetat (je 250 ml) extrahiert. Die vereinigten organischen Extrakte werden mit gesättigter Kochsalzlösung gewaschen und über Natriumsulfat getrocknet. Nach Abziehen des Lösungsmittels verbleiben schwach gelbe Kristalle. Die Umkristallisation erfolgt aus Dimethylformamid (DMF)/Wasser.
Schmelzpunkt: 94.5 - 95.0 ° C
Ausbeute: 74%
$^{1}$H-NMR-Daten in DMSO/TMS
1.4 ppm (s,3H,Me); 3.5 ppm (s,2H,$CH_2$Ar); 3.8 ppm (s,6H,MeOOC); 7.2 ppm (d,2H, ArH); 8.2 ppm (d, 2H,ArH)

2-Methyl-2-(4-nitrobenzyl)-malonsäurediamid [2]

In einen 500 ml Rundkolben werden 5.0 g des Esters [1] eingewogen und in 200 ml Methanol gelöst. In diese methanolische Lösung wird bis zur Sättigung Ammoniak eingeleitet, anschließend die katalytische Menge Natrium zugegeben und nach Beendigung der Gasentwicklung der Kolben mit einem Absaugstück mit Luftballon verschlossen und mindestens 1 Woche bei Raumtemperatur stehen gelassen. Es fällt nach einigen Tagen ein weißer Niederschlag aus. Nachdem sämtliches Edukt umgesetzt ist (DC-Kontrolle), wird der ausgefallene Niederschlag abgesaugt und die verbleibende Lösung im Tiefkühlschrank auf -20 ° C abgekühlt und erneut abgesaugt. Die Mutterlauge wird am Rotationsverdampfer weiter eingeengt und der

ausgefallende Niederschlag wieder abgesaugt. Die Umkristallisation der vereinigten Kristallfraktionen erfolgt aus Acetonitril. Das weiße kristalline Produkt wird im Vakuumexsikkator getrocknet.
Schmelzpunkt: 179°C
Ausbeute: 91%
$^1$H-NMR-Daten in DMSO/TMS
1.2 ppm (s,3H,Me); 3.3 ppm (s,2H,$CH_2$Ar); 7.1 ppm (s(br),4H,$CONH_2$); 7.3 ppm (d,2H, ArH); 8.1 ppm (d,2H,ArH)

2-Methyl-2-(4-nitrobenzyl)-1,3-propandiamin [3]

Das Diamid [2] (5.0 g) wird unter Ausschluß von Luftfeuchtigkeit in 25 ml abs. Tetrahydrofuran in einem 250 ml Zweihalskolben mit Rückflußkühler und Trockenrohr sowie einem Septum suspendiert. Der Kolben wird in einem Eisbad heruntergekühlt und anschließend die Boran-Lösung in THF mit Hilfe einer 20 bzw. 50 ml-Einwegspritze durch das Septum zugegeben. Nachdem die erforderliche Menge der Boran-Lösung (80 ml einer 1 M Lösung in THF) zugegeben wurde, läßt man auf Raumtemperatur erwärmen. Nach 30 Minuten wird das Gemisch für 4 Stunden unter Rückfluß gekocht. Nach Abkühlung auf Raumtemperatur wird der Überschuß an Boran mit Wasser vorsichtig hydrolisiert (insgesamt 16 ml Wasser). Nach Beendigung der Gasentwicklung wird das Redaktionsgemisch quantitativ in einen 500 ml Rundkolben überführt und das Lösungsmittel am Rotationsverdampfer vorsichtig abgezogen, es verbleibt ein weißer Niederschlag. Dieser Niederschlag wird mit 125 ml 6 N HCl versetzt und 3 Stunden unter Rückfluß gekocht. Anschließend wird die Salzsäure am Rotationsverdampfer langsam abgezogen. Der Rückstand wird in der minimalen Menge Wasser aufgenommen (ca. 50-70 ml) und auf eine präparierte Ionenaustauschersäule (stark basisch) gegeben und das reine Amin mit destilliertem Wasser eluiert. Das freie Diamin wird aufgefangen (pH-Kontrolle) und die vereinigten Fraktionen werden am Rotationsverdampfer eingeengt. Als Rückstand verbleibt ein schwach gelbes Öl.
Schmelzpunk (Hydrochlorid): 270°C
Ausbeute: 80%
$^1$H-NMR-Daten in DMSO/TMS
1.0 ppm (s,3H,Me); 2.9 ppm (m,4H,$CH_2NH_2$); 3.0 ppm (s,2H,$CH_2$Ar); 7.5 ppm (d,2H,ArH); 8.2 ppm (d,2H,ArH); 8.5 ppm (s(br),4H,$NH_2$)

2-Methyl-2-(4'-nitrobenzyl)-N,N'-propylen-bis[(5-sulfo)salicylidenamin][4]

Eine Lösung von 5,6 g Diamin [3] in 100 ml 50% Ethanol wird mit Essigsäure auf pH 5-6 eingestellt. Zu dieser Lösung werden 8,6 g 5-Sulfosalicylaldehyd und 1,9 g Natriumcyanoborhydrid gegeben und über Nacht gerührt. Nach Abkühlen auf 0°C wird mit halbkonzentrierter Salzsäure auf pH 2 eingestellt und eingeengt. Der Rückstand wird mit gesättigter Kaliumcarbonatlösung neutralisiert und anschließend lyophilisiert. Der Rückstand wird mit DMF/MeOH extrahiert. Nach Abziehen des Lösungsmittels verbleibt ein kristalliner Rückstand, der aus Acetonitril/Wasser umkristallisiert wird.
Ausbeute: 56%
$^1$H-NMR-Daten in DMSO/TMS
0,9 ppm (s,3H,Me); 2,5 ppm (m,4H,$CH_2$NH); 2,8 ppm (s,2H,$CH_2$Ar); 4,1 ppm (m,4H,Ar$CH_2$NH); 7,0-7,8 ppm (m,8H,ArH); 8,2 ppm (d,2H,ArH)

2-Methyl-2-(4'-aminobenzyl)-N,N'-propylen-bis[(5-sulfo)salicylidenamin][5]

In einem 100 ml Zweihalskolben werden 40 mg 10% Pd/C in 50 ml MeOH suspendiert und mit Wasserstoff gesättigt. Anschließend wird die Lösung von 595 mg [4] (1 mmol) in 5 ml Methanol und 1,6 ml 6N Salzsäure mit einer Einwegspritze durch ein Septum zugegeben. Nach Beendigung der Wasserstoffaufnahme wird vom Katalysator abgetrennt und das Lösungsmittel im Vakuum abgezogen. Es verbleiben weiße Kristalle. Die Umkristallisation erfolgt aus Methanol.
Ausbeute: 76%
$^1$H-NMR-Daten in DMSO/TMS
1,0 ppm (s,3H,Me); 2,5 ppm (m,4H,$CH_2$NH); 2,8 ppm (s,2H,$CH_2$Ar); 4,2 ppm (m,4H,Ar$CH_2$NH); 7,0-7,8 ppm (m,10H,ArH)

2-Methyl-2-(4-isothiocyanatobenzyl)-N,N'-propylen-bis-[(5-sulfo)salicylidenamin][6]

Eine 85%ige Lösung von Thiophosgen in Tetrachlorkohlenstoff (0,2 ml, 2,23 mmol) wird zu der Lösung von 280 mg [5] (0,5 mmol) in 4 ml Salzsäure (3 M) gegeben. Das Reaktionsgemisch wird 3 Stunden bei Raumtemperatur gerührt und dann im Vakuum bis zur Trockene eingeengt.
Ausbeute: 78%
$^1$H-NMR-Daten in DMSO/TMS
1,0 ppm (s,3H,Me); 2,8 ppm (m,4H,$CH_2$NH); 3,0 ppm (s,2H,$CH_2$Ar); 4,2 ppm (m,4H,Ar$CH_2$NH); 7,0-7,8 ppm (m,10H,ArH)

**Beispiel 2:**

2-(4-Nitrobenzyl)-malonsäurediethylester [7]

Es werden 21.4 g Lithiumdiisopropylamid zu 210 ml wasserfreiem Tetrahydrofuran unter einem Stickstoffstrom in einem trockenen Dreihalskolben mit Trockenrohr und Tropftrichter mit Druckausgleich gegeben. Anschließend werden bei Raumtemperatur 58.0 g Malonsäurediethylester in 100 ml wasserfreiem Tetrahydrofuran innerhalb von 40 Minuten zugetropft. Nach 30 Minuten wird die Reaktionslösung auf -62 °C abgekühlt und 39.1 g 4-Nitrobenzylbromid in Tetrahydrofuran langsam unter starkem Rühren zugetropft, eine weitere Stunde bei - 62 °C gerührt und anschließend wird der gebildete Niederschlag in der Kälte abfiltriert. Das Filtrat wird am Rotationsverdampfer eingeengt, der Rückstand in 300 ml Ethanol bei 65 °C in Lösung gebracht und vom unlöslichen Rückstand abgetrennt. Nach dem Abkühlen erhält man 34.7 g schwach gelbliche Kristalle.
Ausbeute: 65%
$^1$H-NMR-Daten in $CDCl_3$/TMS
1.2 ppm (t,6H,$CH_2CH_3$); 3.3 ppm (d,2H,$CH_2$Ar); 3.6 ppm (t,1H,CH); 4.1 ppm (q,4H,$CH_2CH_3$); 7.4 ppm (d,2H,ArH); 8.1 ppm (d,2H, ArH)

2-(4-Nitrobenzyl)-malonsäurediamid [8]

Diese Verbindung wird entsprechend der Verbindung [2] dargestellt.
Ausbeute 98%
$^1$H-NMR-Daten in DMSO/TMS
3.1 ppm (d,2H,$CH_2$Ar); 3.4 ppm (t,1H,CH); 7.1 ppm (s,4H,$NH_2$); 7.4 ppm (d,2H,ArH); 8.2 ppm (d,2H,ArH)

2-(4-Nitrobenzyl)-1,3-propandiamin [9]

Diese Verbindung wird entsprechend der Verbindung [3] dargestellt.
Ausbeute: 76%
$^1$H-NMR-Daten in $D_2O$
1.3 ppm (t,1H,CH); 3.1 ppm (d,4H,$CH_2NH_2$); 3.2 ppm (d,2H,$CH_2$Ar); 7.5 ppm (d,2H,ArH); 8.2 ppm (d,2H,ArH)

2-(4'-Nitrobenzyl)-N,N'-propylen-bis-[(5-sulfo)salicylidenimin [10]

Zu einer Lösung von 5,6 g Diamin [9] in 75 ml Ethanol abs. wird unter Rühren eine Lösung von 9,5 g 5-Sulfosalicylaldehyd in 75 ml Ethanol abs. zugetropft. Die resultierende Lösung verfärbt sich langsam intensiv gelb. Es wird noch 3 Stunden bei Raumtemperatur gerührt, dabei fällt ein gelber Niederschlag aus, der nach Abkühlen auf -20 °C abfiltriert wird.
Ausbeute: 68%
$^1$H-NMR-Daten in DMSO/TMS
1.5 ppm (m,1H,CH); 3,2 ppm (d,2H,$CH_2$Ar); 3,5 ppm (m,4H,$CH_2$N = C); 7.1-7,9 ppm (m,8H,ArH); 8,2 ppm (d,2H,ArH); 8,6 ppm (s,2H,CH = N)

2-(4'-Nitrobenzyl)-N,N'-propylen-bis-[(5-sulfo)salicylidenamin] [11]

Zu einer Lösung von 2 g Diimin [10] in 50 ml 50% Ethanol werden unter Rühren 500 mg Natriumborhydrid zugegeben und 4 Stunden bei 0 °C gerührt. Anschließend werden 20 ml Wasser zugetropft und 1 Stunde bei Raumtemperatur gerührt, danach das Lösungsmittel am Rotationsverdampfer abgezogen und mit einer gesättigten Kaliumcarbonatlösung auf pH 11 gebracht. Der Rückstand wird in Methanol aufgenommen und über eine kurze Säule (RP-18) filtriert. Nach Trocknen über Natriumsulfat und abziehen des Lösungsmittels verbleibt ein kristalliner Rückstand.
Ausbeute: 80 %
$^1$H-NMR-Daten in DMSO/TMS
1.6 ppm (m,1H,CH); 2,5 ppm (m,4H,$CH_2$NH); 2,9 ppm (d,2H,$CH_2$Ar); 4,0 ppm (m,4H,Ar$CH_2$NH); 7,1-7,8 ppm (m,8H, ArH); 8,2 ppm (d,2H,ArH)

Cu-2-(4'-Nitrobenzyl)-N,N'-propylen-bis-[(5-sulfo)salicyliden-amin] [12]

Zu einer Suspension von 1,63 g [11] (2 mmol) in 50 ml Methanol werden 400 mg Kupferacetat (2 mmol) in 20 ml Methanol getropft und bei Raumtemperatur gerührt. Nach Abziehen des Lösungsmittels und Trocknen verbleibt ein kristalliner Rückstand.
Ausbeute: 66%

Cu-2-(4'-Aminobenzyl)-N,N'-propylen-bis[(5-sulfo}salicylidenamin] [13]

Diese Verbindung wird entsprechend der Verbindung [5] dargestellt.
Ausbeute: 78 %

Cu-2-[4'-(Biotincarbamoyl)benzyl]-N,N'-propylen-bis[(5-sulfo)salicylidenamin] [14]

Zu einer Lösung von 615 mg [13] (1 mmol) in 10 ml DMSO werden 680 mg NHS-Biotin (2 mmol) unter Rühren dazugegeben und 3 Stunden auf 50 ° C erwärmt. Nach Abkühlen auf Raumtemperatur wird noch 15 Stunden gerührt. Anschließend wird das Lösungsmittel im Vakuum abgezogen und der Rückstand mittels MPLC gereinigt (Kieselgel, Dichlormethan/Ethanol/ konzentriertes Ammoniak 10:20:1)
Ausbeute: 38%

2-[4'-(Biotincarbamoyl)benzyl]-N,N'-propylen-bis[{5-sulfo)salicylidenamin] [15]

Zu einer Lösung von 500 mg [14] in 70 ml Wasser werden bei 40 ° C 500 mg Kaliumcyanid zugegeben und noch 2 Stunden gerührt. Anschließend wird auf ca. 10 ml eingeengt und der Rückstand mittels MPLC gereinigt (Kieselgel, Acetonitril/konzentriertes Ammoniak 40:3).
Ausbeute: 46%
$^1$H-NMR-Daten in DMSO/TMS
1,3-1,7 ppm (m,7H,$CH_2$ + CH); 2,3 ppm (t,2H,$CH_2$CO); 2,8 ppm (m,2H, $CH_2$S); 3,0 ppm (s,2H,$CH_2$Ar); 3,1 ppm (m,1H, CHS); 4,1 ppm (m,1H, CHNHCO); 4,3 ppm (m,5H,Ar$CH_2$NH + CHNHCO); 6,9 ppm-7,8 ppm (m,10H,ArH).

**Beispiel 2a**

Tc-99m-Komplex von [15]

Zu einer Lösung von 1,86 * $10^{-6}$ mol [15] in 1 ml EtOH/$H_2O$ 1:9 werden 5,6 * $10^{-3}$ mol Kaliumtartrat in 1 ml $H_2O$, 4,8 * $10^{-8}$ mol Zinnchlorid in 1 ml $H_2O$ und 5 mCi Tc-99m-Pertechnetat gegeben und 10 Minuten inkubiert. Der entsprechende Technetium-Komplex wird in einer Reinheit >90% erhalten.

**Beispiel 3**

2-(4'-Nitrobenzyl)-N,N'-propylen-bis-[(5-carboxy)salicylidenamin [16]

Diese Verbindung wird entsprechend der Verbindung [4] dargestellt.
Ausbeute: 66%
$^1$H-NMR-Daten in DMSO/TMS
0,9 ppm (s,3H,Me); 2,5 ppm (m,4H,$CH_2$NH); 2,8 ppm (s,2H,$CH_2$Ar); 4,2 ppm (m,4H,Ar$CH_2$NH): 6,9-8,0 ppm (m,8H,ArH); 8,2 ppm (d,2H,ArH)

Cu-2-(4'-Nitrobenzyl)-N,N'-propylen-bis-[(5-carboxy)salicyliden-amin] [17]

Diese Verbindung wird entsprechend der Verbindung [12] dargestellt.
Ausbeute: 74%

Cu-2-(4'-Aminobenzyl)-N,N'-propylen-bis[5-(carboxy)salicylidenamin [18]

Diese Verbindung wird entsprechend der Verbindung [13] dargestellt.
Ausbeute: 69%

Cu-2-[4'-(Biotincarbamoyl)benzyl]-N,N'-propylen-bis[(5-carboxy)salicylidenamin] [19]

Diese Verbindung wird entsprechend der Verbindung [14] dargestellt.
Ausbeute: 43%

2-[4'-(Biotincarbamoyl)benzyl]-N,N'-propylen-bis[(5-carboxy)salicylidenamin] [20]

Diese Verbindung wird entsprechend der Verbindung [15] dargestellt.
Ausbeute: 49%
$^1$H-NMR-Daten in DMSO/TMS
1,3-1,7 ppm (m,7H,$CH_2$ + CH); 2,3 ppm (t,2H,$CH_2$CO); 2,8 ppm (m,2H, $CH_2$S); 3,0 ppm (s,2H,$CH_2$Ar); 3,1 ppm (m,1H, CHS); 4,1 ppm (m,1H, CHNHCO); 4,2 ppm (m,5H,Ar$CH_2$NH + CHNHCO): 6,9 ppm-8,1 ppm (m,10H,ArH).

**Beispiel 4**

2-Brommethyl-5-nitrobenzoesäure [21]

Zu 72 g 2-Methyl-5-nitrobenzoesäure und 100 mg Dibenzoylperoxid in 250 ml Tetrachlorkohlenstoff werden unter Bestrahlung mit einer 500 W Glühlampe 20 ml Brom langsam zugetropft. Die Reaktionsmischung wird 24 Stunden unter Rückfluß erhitzt, nach Abkühlen das Lösungsmittel abgezogen, in Dichlormethan aufgenommen, mehrmals mit Wasser gewaschen, getrocknet und eingeengt. Der Rückstand wird chromatographisch gereinigt (Kieselgel, Dichlormethan).
Ausbeute: 56%
$^1$H-NMR-Daten in $CDCl_3$/TMS
4,4 ppm (s,2H,$CH_2$); 8,3-8,5 ppm (m,3H,ArH).

(2-Carboxy-4-nitrobenzyl)malondinitril [22]

Zu einer Suspension von 11,9 g Natriumhydrid in 100 ml wasserfreiem DMF wird die Lösung von 16,4 g Malondinitril in 100 ml wasserfreiem DMF bei 0 °C innerhalb von 3 Stunden zugetropft. Anschließend werden 65 g [21] in 100 ml wasserfreiem DMF innerhalb von 6 Stunden zugetropft und nach Erwärmen auf Raumtemperatur noch weitere 12 Stunden gerührt. Das Reaktionsgemisch wird mit Eiswasser hydrolisiert, angesäuert und mit Chloroform extrahiert. NachTrocknen und Einengen verbleibt kristalliner Rückstand, der aus Methanol umkristallisiert wird.
Ausbeute: 48%
$^1$H-NMR-Daten in $CDCl_3$/TMS
3,5 ppm (d,2H,$CH_2$Ar); 3,8 ppm (t,1H,CH); 8,3-8,5 ppm (m,3H,ArH).

2-(2-Carboxy-4-nitrobenzyl)-1,3-propandiamin [23]

Zu einer Lösung von 12 g [22] (0,05 mol) und 24 g Cobaltchlorid Hexahydrat (0,1 mol) in 300 ml 99% Methanol werden bei -15 °C 19g Natriumborhydrid langsam zugegeben und anschließend noch 3 Stunden gerührt. Nach Zugabe von 100 ml 3N Salzsäure wird das Lösungsmittel größtenteils abdestilliert, die verbleibende wässrige Phase mit gesättigter Kaliumcarbonat-Lösung alkalisiert und mehrmals mit Dichlormethan extrahiert. Nach Trocknen und Einengen verbleibt ein Rückstand, der aus Ethanol umkristallisiert wird.
Ausbeute: 59%
$^1$H-NMR-Daten in DMSO
1,3 ppm (t,1H,$CH_2$); 3.1 ppm (d,2H,$CH_2NH_2$); 3,4 ppm (d,2H,$CH_2$Ar); 8.3-8,5 ppm (m,3H,ArH)

2-[2'-Carboxy-(4'-nitrobenzyl)]-N,N'-propylen-bis-salicylidenamin [24]

Diese Verbindung wird entsprechend der Verbindung [4] dargestellt.
Ausbeute: 62%
$^1$H-NMR-Daten in $CDCl_3$/TMS
1.6 ppm (m,1H,CH); 2,5 ppm (m,4H,$CH_2$NH); 3,0 ppm (d,2H,$CH_2$Ar); 3,9 ppm (m,4H,Ar$CH_2$NH); 6,9 ppm (m,4H,ArH); 7,1-7,3 ppm (m,4H, ArH); 8,3-8,4 ppm (m,3H,ArH)

2-[2'-Carboxy-(4'-aminobenzyl)]-N,N'-propylen-bis-salicylidenamin [25]

Diese Verbindung wird entsprechend der Verbindung [5] dargestellt.
Ausbeute: 85%
$^1$H-NMR-Daten in DMSO/TMS
1.5 ppm (m,1H,CH); 2,5 ppm (m,4H,$CH_2NH_2$); 2,9 ppm (t,2H,$CH_2$Ar); 4,1 ppm (m,4H,Ar$CH_2$NH); 6,8-7,4 ppm (m,11H,ArH)

2-[2'-Carboxy-(4'-isothiocyanatobenzyl)]-N,N'-propylen-bis-salicylidenamin [26]

Diese Verbindung wird entsprechend der Verbindung [6] dargestellt.
Ausbeute: 77%
$^1$H-NMR-Daten in DMSO/TMS
1,5 ppm (m,1H,CH); 2,9 ppm (m,5H,$CH_2$NH + $CH_2$Ar)); 3,0 ppm (s,2H, $CH_2$Ar); 4,1ppm (m,4H,Ar$CH_2$NH); 6,8-7,4 ppm (m,11H,ArH).

**Beispiel 5**

6-(2'-Carboxy-4'-nitrobenzyl)-3,3,9,9-tetramethyl-4,8-diazaundecan-2,10-dion-dioxim [27]

Es werden 13 g Diamin [23] in 90 ml Methanol gelöst und auf 0 °C abgekühlt, anschließend werden unter Rühren 6.5 g 2-Chlor-2-methyl-3-nitrosobutan zugegeben. Nach Erwärmen auf Raumtemperatur wird 2 Stunden gerührt, weitere 2 Stunden unter Rückfluß gekocht, das Lösungsmittel abgezogen und der verbleibende Festkörper in Wasser gelöst. Es wird mit Natriumhydrogencarbonat neutralisiert, eingeengt und der verbleibende Rückstand aus Ethanol umkristallisiert.
Ausbeute: 38%
$^1$H-NMR-Daten in DMSO/TMS
1.3 ppm (s,12H,MeCNH); 1.8 ppm (s,6H,MeC = N); 2.4 ppm (m,1H,CH); 2.5 ppm (m,4H,$CH_2$NH); 2.9 ppm (d,2H,$CH_2$Ar); 8.2-8.4 ppm (m,3H,ArH); 10.8 ppm (s,2H,HON = C)

6-(2'-Carboxy-4'-aminobenzyl)-3,3,9,9-tetramethyl-4,8-diazaundecan-2,10-dion-dioxim [28]

Es werden 500 mg der Nitroverbindung [27] in 50ml 50%igem Methanol (pH 11) gelöst und bei Raumtemperatur unter Zusatz von 25 mg Pd/Alox hydriert. Nach Abtrennen des Katalysators und Einengen verbleibt ein kristalliner Festkörper.
Ausbeute: 49%
$^1$H-NMR-Daten in $D_2O$
1.2 ppm (s,12H,MeCNH); 1.7 ppm (s,6H,MeC = N); 1.9 ppm (m,1H,CH); 2.5 ppm (m,4H,$CH_2$NH); 2.5 ppm (d,2H,$CH_2$Ar); 6.7 ppm (d,1H,ArH); 7,3 ppm (m,2H,ArH)

Cu-6-(2'-Carboxy-4'-aminobenzyl)-3,3,9,9-tetramethyl-4,8-diazaundecan-2,10-dion-dioxim [29]

Diese Verbindung wird entsprechend der Verbindung [12] dargestellt
Ausbeute: 58%

Cu-6-[2'-carboxy-4'-(biotincarbamoyl)benzyl]-3,3,9,9-tetramethyl-4,8-diazaundecan-2,10-dion-dioxim [30]

Diese Verbindung wird entsprechned der Verbindung [14] dargestellt.
Ausbeute: 34%

6-(2'-Carboxy-4'-biotincarbamoyl)benzyl]-3,3,9,9-tetramethyl-4,8-diazaundecan-2,10-dion-dioxim [31]

Diese Verbindung wird entsprechned der Verbindung [15] dargestellt.
Ausbeute: 41%

Tc-99m-Komplex von [31]

Zu einer Lösung von 1,86 * $10^{-6}$ mol [31] in 1 ml EtOH/$H_2O$ 1:4 werden 3,2 * $10^{-3}$ mol Kaliumtartrat in 1 ml $H_2O$, 4,0 * $10^{-8}$ mol Zinnchlorid in 1 ml $H_2O$ und 5 mCi Tc-99m-Pertechnetat gegeben und 10 Minuten inkubiert. Der entsprechende Technetium-Komplex wird in einer Reinheit >90% erhalten.

**Beispiel 6**

2-Methyl-2-[4'-nitrobenzoyl]-malonsäuredimethylester [32]

Zu der Mischung aus 24,3 g Magnesiumspänen (1 mol) in 50 ml wasserfreiem Ethanol werden 5 ml Tetrachlorkohlenstoff gegeben. Anschließend wird eine Lösung von 174 g Methylmalonsäurediethylester (1 mol) in 100 ml Ethanol und 400 ml wasserfreiem Ether so zugetropft, daß die Mischung kräftig siedet. Nach Auflösen des Magnesiums wird unter Eiskühlung 185 g 4-Nitrobenzoylchlorid in 100 ml wasserfreiem Ether zugetropft und noch 12 Stunden gerührt. Unter Eiskühlung wird hydrolisiert, die Etherphase abgetrennt, mehrmals mit Ether extrahiert, neutral gewaschen, getrocknet und eingeengt. Nach Destillation im Vakuum wird das Reinprodukt erhalten.
Ausbeute: 78%
$^1$H-NMR-Daten in $CDCl_3$/TMS
1,2 ppm (m,9H,Me); 4,1 ppm (q,4H,$CH_2$Me); 8,1-8,3 ppm (m,4H,ArH)

2-Methyl-2-[4'-nitrobenzoyl]-malonsäure [33]

Es werden 41 g [32] (127 mmol) in 200ml Methanol gelöst und dazu eine Lösung von 28.3 g NaOH (708 mmol) in 50 ml Wasser langsam zugetropft. Anschließend wird 2 Stunden im Wasserbad auf 50°C erwärmt, das Lösungsmittel am Rotationsverdampfer abgezogen und der Rückstand in Wasser aufgenommen. Es wird mit halbkonzentrierter Salzsäure angesäuert und die freie Carbonsäure mit Chloroform extrahiert, gewaschen und getrocknet.
Ausbeute: 80%
$^1$H-NMR-Daten in DMSO/TMS
1,2 ppm (s,3H,Me); 8,1-8,3 ppm (m,4H,ArH)

2-Methyl-2-[4'-nitrobenzoyl]-malonsäurechlorid[34]

Die Mischung aus 3.40 g [33] und 8.9 g Thionylchlorid und einem Tropfen DMF wird unter Feuchtigkeitsausschluß unter Rühren zum Sieden erhitzt. Nach Beendigung der Gasentwicklung wird der Überschuß Thionylchlorid bei Raumtemperatur im Vakuum abgezogen und der Rückstand im Vakuum destilliert.
Ausbeute: 91%

N,N'-Bis[(2-(benzylthio)benzyl]-2-methyl-2-(4'-nitrobenzoyl)malonsäurediamid [35]

Zu dem Gemisch aus 10,5 g (2-Mercaptobenzyl)benzylamin (48,6 mmol) und 5,06 g Triethylamin (50 mmol) in 100 ml Dichlormethan wird unter Ausschluß von Feuchtigkeit 7,4 g [34] (24,3 mmol) in 100 ml Dichlormethan vorsichtig zugetropft. Es wird noch 3 Stunden bei Raumtemperatur gerührt, dann zunächst Wasser zugegeben und mehrmals mit Chloroform extrahiert. Die organische Phase wird nacheinander mit 1N HCl, gesättigter Kaliumcarbonatlösung und Wasser gewaschen und über Natriumsulfat getrocknet.
Ausbeute: 64%
$^1$H-NMR-Daten in $CDCl_3$/TMS
1,2 ppm (s,3H,Me); 4,1 ppm (s,8H,Ar$CH_2$S + Ar$CH_2$NH); 7,1-7,6 ppm (m,18H,ArH); 8,3 ppm (m,4H,ArH).

N,N'-Bis[(2-benzylthio)benzyl]-2-methyl-2-[hydroxy-(4-nitrophenyl)methyl)]-malonsäurediamid [36]

Zu einer Lösung von 66 g (100 mmol) [35] in 50 ml Ethanol werden 5,7 g Natriumborhydrid gegeben

und 2 Stunden bei Raumtemperatur gerührt. Es wird weitgehend eingedampft und der Rückstand mit 50 ml Wasser versetzt und neutralisiert. Es wird mit Ether extrahiert, mit gesättigter Kochsalzlösung gewaschen, getrocknet und eingeengt.
Ausbeute: 77%
$^1$H-NMR-Daten in DMSO/TMS
1,4 ppm (s,3H,Me); 4,1 ppm (s,8H,$ArCH_2S + ArCH_2NH$); 4,3 ppm (m,1H,CHOH); 7,1-7,6 ppm (m,20H,ArH); 8,2 ppm (m,2H,ArH).

N,N'-Bis[(2-benzylthio)benzyl]-2-methyl-2-[hydroxy-(4-nitrophenyl)methyl)]-1,3-propandiamin [37]

Diese Verbindung wird entsprechend der Verbindung [3] dargestellt.
$^1$H-NMR-Daten in $CDCl_3$/TMS
1,5 ppm (s,3H,Me); 2,5 ppm (m,4H,$CH_2NH$); 4,1 ppm (s,4H,$ArCH_2NH$); 4,3 ppm (m,1H,CHOH); 7,0-7,5 ppm (m,10H,ArH); 8,2 ppm (d,1H,ArH).

N,N'-Bis(2-mercaptobenzyl)-2-methyl-2-[hydroxy-(4-nitrophenyl)methyl)]-1,3-propandiamin [38]

Zu einer auf -50 °C gekühlten Lösung von 4,3 g [37] in 50 ml THF und 100 ml flüssigem Ammoniak werden 3,5 g Natrium vorsichtig zugegeben und rührt 4 Stunden. Überschüssiges Natrium wird vorsichtig mit Ammoniumchlorid zerstört und läßt anschließend langsam auf Raumtemperatur erwärmen. Der Rückstand wird in Wasser aufgenommen und mehrmals mit Dichlormethan extrahiert, die organische Phase getrocknet und eingeengt.
Ausbeute: 62%
$^1$H-NMR-Daten in $CDCl_3$/TMS
1,5 ppm (s,3H,Me); 2,5 ppm (m,4H,$CH_2NH$); 4,1 ppm (s,4H,$ArCH_2NH$); 4,3 ppm (m,1H,CHOH); 7,0-7,5 ppm (m,10H,ArH); 8,2 ppm (d,1H,ArH).

N,N'-Bis(2-mercaptobenzyl)-2-methyl-2-[hydroxy-(4-aminophenyl)methyl)]-1,3-propandiamin [39]

Diese Verbindung wird entsprechend der Verbindung [5] dargestellt.
Ausbeute: 89%
$^1$H-NMR-Daten in $CDCl_3$/TMS
1,5 ppm (s,3H,Me); 4,1 ppm (s,4H,$ArCH_2NH$); 4,3 ppm (m,1H,CHOH); 7,0-7,7 ppm (m,11H,ArH);

N,N'-Bis(2-mercaptobenzyl)-2-methyl-2-[hydroxy-(4-isothiocyanatophenyl)methyl]-1,3-propandiamin [40]

Diese Verbindung wird entsprechend der Verbindung [6] dargestellt.
Ausbeute: 49%
$^1$H-NMR-Daten in $CDCl_3$/TMS
1,5 ppm (s,3H,Me); 4,1 ppm (s,4H,$ArCH_2NH$); 4,2 ppm (m,1H,CHOH); 7,0-7,6 ppm (m,11H,ArH);

**Beispiel 7**

N,N'-Bis[(2-benzylthio-3-pyridyl)methyl]-2-methyl-2-(4'-nitrobenzyl)malonsäurediamid [41]

Diese Verbindung wird entsprechend der Verbindung [35] dargestellt.
Ausbeute: 60%
$^1$H-NMR-Daten in $CDCl_3$/TMS
1,2 ppm (s,3H,Me); 3,5 ppm (s,2H,$CH_2Ar$); 4,1 ppm (s,8H,$ArCH_2S + ArCH_2NH$); 7,1-7,5 ppm (m,14H,ArH); 8,3-8,5 ppm (m,6H,ArH).

N,N'-Bis[(2-benzylthio-3-pyridyl)methyl]-2-methyl-2-(4'-nitrobenzyl)-1,3-propandiamin [42]

Diese Verbindung wird entsprechend der Verbindung [3] dargestellt.
Ausbeute: 57%
$^1$H-NMR-Daten in $CDCl_3$/TMS
1,0 ppm (s,3H,Me); 2,5 ppm (m,4H,$CH_2NH$); 2,9 ppm (s,2H,$CH_2Ar$); 4,1 ppm (s,8H,$ArCH_2NH + ArCH_2S$); 7,1-7,5 ppm (m,14H,ArH); 8,3-8,5 ppm (m,6H,ArH).

N,N'-Bis[(2-mercapto-3-pyridyl)methyl]-2-methyl-2-(4-nitrobenzyl)-1,3-propandiamin [43]

Diese Verbindung wird entsprechend der Verbindung [38] dargestellt.
Ausbeute: 65%
$^1$H-NMR-Daten in $CDCl_3$/TMS
1,0 ppm (s,3H,Me); 2,5 ppm (m,4H,$CH_2$NH); 2,9 ppm (s,2H,$CH_2$Ar); 4,1 ppm (s,4H,Ar$CH_2$NH); 7,0-7,5 ppm (m,4H,ArH); 8,2-8,5 ppm (m,6H,ArH).

N,N'-Bis[(2-mercapto-3-pyridyl)methyl]-2-methyl-2-(4-aminobenzyl)-1,3-propandiamin [44]

Diese Verbindung wird entsprechend der Verbindung [5] dargestellt.
Ausbeute: 89%
$^1$H-NMR-Daten in $CDCl_3$/TMS
1,0 ppm (s,3H,Me); 2,5 ppm (m,4H,$CH_2$NH); 2,8 ppm (s,2H,$CH_2$Ar); 4,1 ppm (s,4H,Ar$CH_2$NH); 7,0-7,7 ppm (m,6H,ArH); 8,3-8,5 ppm (m,4H,ArH).

N,N'-Bis[(2-mercaptopyridyl)methyl]-2-methyl-2-(4-isothiocyanatobenzyl)-1,3-propandiamin [45]

Diese Verbindung wird entsprechend der Verbindung [6] dargestellt.
Ausbeute: 46%
$^1$H-NMR-Daten in $CDCl_3$/TMS
1,0 ppm (s,3H,Me); 2,5 ppm (m,4H,$CH_2$NH); 2,8 ppm (s,2H,$CH_2$Ar); 4,1 ppm (s,4H,Ar$CH_2$NH); 7,0-7,7 ppm (m,6H,ArH); 8,3-8,5 ppm (m,4H,ArH).

**Beispiel 8**

Kopplung und Markierung eines Isothiocyanat-haltigen Chelatbildners an Proteine

Am Beispiel von $F(ab)_2$-Fragmenten des monoklonalen Antikörpers 17-1A soll die Kopplung von Isothiocyanat-haltigen Tc-Chelatbildnern Verbindung [6] an Proteine beschrieben werden. Anstelle der Antikörper-Fragmente kann jedes andere Protein bzw. eine Aminogruppen-haltige Substanz verwendet werden.

Der monoklonale Antikörper 17-1A wird entsprechend literaturbekannter Methoden nach Applikation von $10^7$ der entsprechenden Hybridomazellen in den Bauchraum einer Balb/c-Maus und Aspiration der Ascitesflüssigkeit nach 7 - 10 Tagen gewonnen. Die Reinigung erfolgt nach ebenfalls literaturbekannten Methoden durch Ammoniumsulfatfällung und Affinitätschromatographie über Protein A-Sepharose. Der gereinigte Antikörper(10 mg/ml) wird bei pH 3,5 für 2 Std. mit 25 µg/ml Pepsin behandelt und anschließend mittels FPLC isoliert. Vor der Kopplung mit dem Chelatbildner werden die Fragmente bei 4 °C für 12 - 24 Std. gegen 0,1 M $KH_2PO_4$/0,1 M $NaHCO_3$, pH 8,5, dialysiert. Die Proteinkonzentration wird auf 10 mg/ml eingestellt. Ein 5facher molarer Überschuß des NCS-haltigen Chelatbildners [Beispiel 1] wird in möglichst wenig des gleichen Puffers gelöst und zur Proteinlösung hinzugegeben. Zur Konjugatbildung wird die Mischung für 3 Std. bei 37 °C inkubiert. Anschließend wird das Konjugat für 24 -48 Std. mit mehrfachem Pufferwechsel gegen PBS (Phosphat-gepufferte Saline) dialysiert und die Proteinkonzentration danach nötigenfalls wieder auf 10 mg/ml eingestellt. Bis zur Markierung mit Tc-99m kann das Konjugat nach Sterilfiltration bei 4 °C in säuregereinigten Glasgefäßen aufbewahrt werden.

Die Markierung von 1 mg des mit dem Chelatbildner [6] gekoppelten Antikörper-Fragments mit Tc-99m erfolgt durch Zugabe von 10 mCi Pertechnetatlösung (= 1 - 2 ml) und 100 µg Zinn-II-chlorid in einer Argon-gespülten Na-Pyrophosphat-Lösung (1mg/ml) oder durch Ligandenaustausch z.B. durch Zugabe der Lösung eines mit Pertechnetat versetzten kommerziell erhältlichen Glucoheptonat-Kits.

**Beispiel 9**

6-(2-Propinyl)-3,3,6,9,9-pentamethyl-4,8-diazaundecan-2,10-dion-dioxim [45]

Es werden bei 0 °C 0.5 g 2-Methyl-2-propinyl-1,3-propandiamin in 8 ml Methanol gelöst, 1.6 g 2-Chlor-2-methyl-3-nitrosobutan und 0.16 g Natriumhydroxid zugegeben. Man rührt 30 Minuten bei dieser Temperatur, erwärmt weitere 10 Stunden auf 60 °C und engt anschließend die Lösung ein. Der verbleibende Festkörper wird an Kieselgel mit Ethanol/Ammoniak chromatographiert sowie aus Isopropanol umkristalli-

siert.
Ausbeute: 53%
$^1$H-NMR-Daten in DMSO/TMS:
1.05 ppm (s,3H, Me); 1.22 ppm (s, 12H, Me); 1.32 ppm (m, 4H, $CH_2$-N); 1.7 ppm (s, 6H, Me); 1.8 ppm (m, 2H, $CH_2$)

3,1β-Dihydroxy-17α-[6,6-bis(2-aza-3,3,4-trimethyl-butan-4-on-oxim}-hept-1-(E)-en-3-in]-1,3,5-estratrien [46]

0.17 g 17β-Jodvinylestradiol [hergestellt nach H. Hofmeister, H. Laurent, P.-E. Schulze und R. Wiechert, Tetrahedron 42, 3575 (1986)] werden mit 0.13g [45], 5mg Benzyltriethylammoniumchlorid, 18.5 mgTetrakis-(triphenylphosphin)-palladium und 14 mg Kupferiodid in 8 ml Toluol gelöst, mit 2 ml 10 %iger Natronlauge versetzt und 72 Stunden bei 30 °C gerührt. Anschließend wird das Lösemittel entfernt und der verbleibende Rückstand an Kieselgel mit Essigsäureethylester/Ethanol chromatographiert.
Ausbeute: 13%
$^1$H-NMR in $CDCl_3$/TMS:
0.95 ppm (s,3H,Me[18]); 1.15 ppm (s,12H,Me); 1.62 ppm (s,6H, Me); 1.20-3.00 ppm (m,15H,Steroid) 1.35 ppm (s,3H,Me); 1.90 ppm (m, 4H, $CH_2$N); 2.75 ppm (d,2H,$CH_2$-CC); 5.45 und 6.2 ppm (AB,2H,CH=CH); 6.65 ppm (d,1H, 4-H); 6.70 ppm (dd,1H, 2-H); 7.15 ppm (d, 1H, 1-H)

**Beispiel 10**

3,17β-Dihydroxy-17α-[6,6-bis-(N,N'-{o-hydroxy}-benzyl-amino-methyl)-hept-1-(E)-en-3-in]-1,3,5-estratrien [47]

0.12 g 17β-Jodvinylestradiol werden mit 0.11 g 2-Methyl-2-(2-propinyl)-N,N'-propylen-bis-(salicylidenamin) [hergestellt nach Europäische Patentanmeldung Nr. 0 417 870 A2], 3.5 mg Benzyltriethylammoniumchlorid, 12 mg Tetrakis-(triphenylphosphin)-palladium und 10 mg Kupferiodid in 5 ml Toluol suspendiert, mit 1.5 ml 10 %iger Natronlauge versetzt und 72 Stunden bei 30 °C gerührt. Anschließend wird das Lösemittel entfernt und an Kieselgel mit Essigsäureethylester/Hexan/Ethanol 2:2:1 chromatographiert.
Ausbeute: 21%
$^1$H-HMR in $CDCl_3$/TMS:
0.93 ppm (s,3H,Me[18]); 1.20-3.00 ppm (m,15H,Steroid); 1.55 ppm (s,3H,Me); 2.06 ppm (m, 4H, $CH_2$N); 2.88 ppm (d,2H,$CH_2$-CC); 4.36 ppm (m,4H,Ar$CH_2$N); 5.40 und 6.20 ppm (AB,2H,CH=CH); 6.60 ppm (d,1H, 4-H); 6.73 ppm (dd,1H, 2-H); 7.10 ppm (d, 1H, 1-H); 6.80 und 7.25 ppm (m,8H, Ar)

## Patentansprüche

1. Verbindungen der allgemeinen Formel I

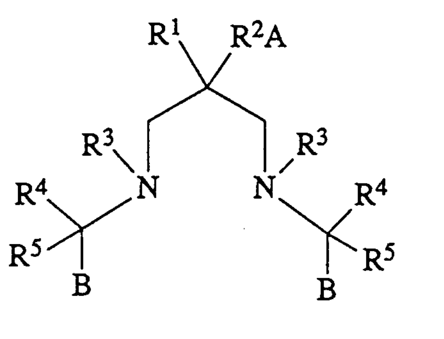

(I)

worin $R^1$ für Wasserstoff oder einen gegebenenfalls mit ein oder zwei Hydroxylgruppen substituierten $C_{1-6}$-Alkylrest,

$R^2$ für einen gegebenenfalls mit einer Hydroxylgruppe substituierten $C_{1-6}$-Alkylenrest,

$R^3$ für ein Wasserstoffatom, einen $C_{1-6}$-Alkyl-, einen Carboxymethyl- oder einen ($C_{1-6}$-Alkoxycarbonyl)methylrest,

$R^4$ für ein Wasserstoffatom, einen gegebenenfalls mit einer Hydroxylgruppe substituierten $C_{1-6}$-Alkylrest bzw. für die unter $R^x$ angegebene Bedeutung steht,

$R^5$ für ein Wasserstoffatom oder einen gegebenenfalls mit einer Hydroxylgruppe substituierten $C_{1-6}$-Alkylrest steht,

B für einen Pyrrolyl-, einen substituierten Phenylrest der Formel II oder einen substituierten Pyridinrest der Formel III

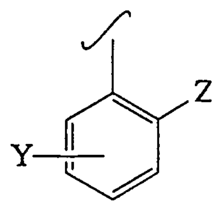 (II) 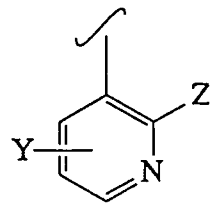 (III)

worin Z eine Hydroxyl-, Amino- oder eine Mercaptogruppe und Y ein Wasserstoffatom, einen Carboxy- oder einen Sulfonylrest bedeuten

oder für einen Nitrosomethylrest der Formel IV

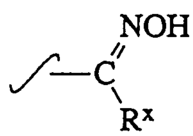 (IV)

steht,

worin $R^x$ einen $C_{1-6}$-Alkylrest, der gegebenenfalls zusammen mit $R^4$ über eine Trimethylen- oder Tetramethylengruppe zu einem 5- bzw. 6-Ring cyclisiert ist, bedeutet

und A für einen Amino-, einen Mercapto-, einen Carboxy-, einen $C_{2-6}$-Alkinyl- oder -Alkenyl-, einen Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht,

wobei die Phenylgruppe gegebenfalls zusätzlich durch einen Carboxy-, einen Chlorsulfonyl- oder einen Sulfonsäurerest substituiert sein kann

oder eine - mit Hilfe der genannten funktionellen Gruppe gegebenenfalls über einen bifunktionellen Linkerrest L gebundene - sich selektiv in Läsionen oder bestimmten Geweben anreichernde Verbindung T enthält,

wobei T für monoklonale Antikörper oder deren Fragmente, Hormone, Enzyme, Liganden für Zellmembranrezeptoren, Neurotransmitter, Lipide, Steroide, Saccharide, Aminosäuren und Oligopeptide, Biotin, sowie Radiosensitizer, wie z.B. Misonidazol steht,

wobei im Falle von

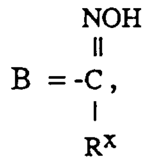

Pyrrolyl- oder Y = H, $R^2$ für eine hydroxilierte $C_{1-6}$-Alkylenkette oder A für einen substituierten Aminophenyl- oder Isothiocyanatophenylrest stehen sollen

sowie deren Komplexe mit- zur Diagnostik und Tumortherapie geeigneten - radioaktiven Metallionen, sowie deren Salze mit anorganischen und organischen Säuren und Basen.

2. Verbindungen nach Anspruch 1, **dadurch gekennzeichnet**, daß der in A gegebenenfalls enthaltene Rest L aus bifunktionellen Linker wie z.B.

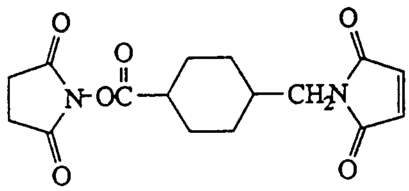
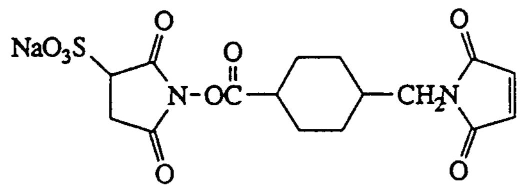

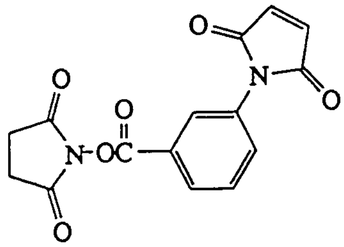
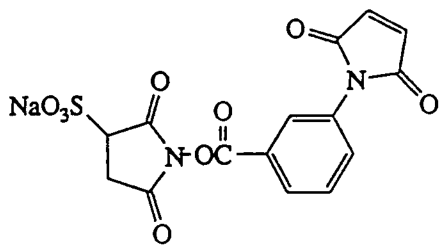

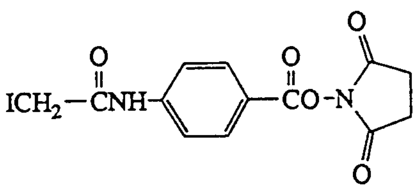
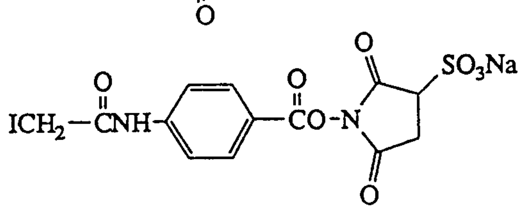

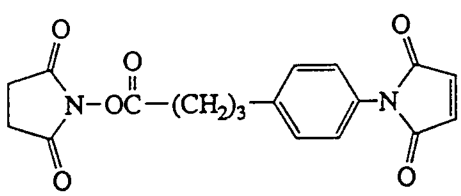
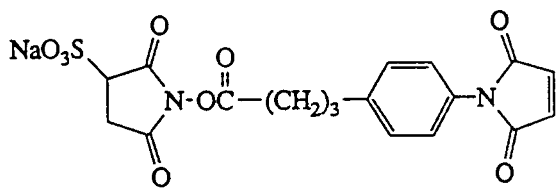

entstanden ist.

3. Verbindungen nach Anspruch 1, **dadurch gekennzeichnet**, daß T für monoklonale Antikörper, deren Fragmente, Biotin oder Misonidazol steht.

4. Metallchelate nach Anspruch 1 mit koordinativ gebundenen, radioaktiven Ionen von Tc, Re, Cu, Co, Ga, Y und In, bevorzugt Tc und Re.

5. Verwendung der Chelate gemäß Anspruch 4 für die In-vivo-Diagnostik und Tumortherapie.

6. Pharmazeutische Mittel enthaltend mindestens ein Chelat gemäß Anspruch 1, gegebenenfalls mit den in der Galenik üblichen Zusätzen.

7. Verfahren zur Herstellung von Verbindungen der allgemeinen Formel I

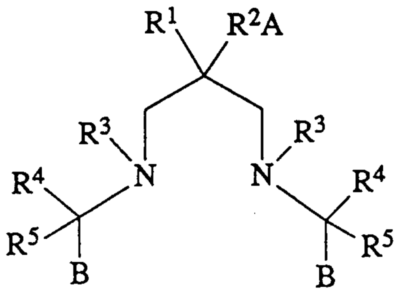

(I)

worin $R^1$ für Wasserstoff oder einen gegebenenfalls mit ein oder zwei Hydroxylgruppen substituierten $C_{1-6}$-Alkylrest,

$R^2$ für einen gegebenenfalls mit einer Hydroxylgruppe substituierten $C_{1-6}$-Alkylenrest,

$R^3$ für ein Wasserstoffatom, einen $C_{1-6}$-Alkyl-, einen Carboxymethyl- oder einen ($C_{1-6}$-Alkoxycarbonyl)methylrest,

$R^4$ für ein Wasserstoffatom, einen gegebenenfalls mit einer Hydroxylgruppe substituierten $C_{1-6}$-Alkylrest bzw. für die unter $R^x$ angegebene Bedeutung steht,

$R^5$ für ein Wasserstoffatom oder einen gegebenenfalls mit einer Hydroxylgruppe substituierten $C_{1-6}$-Alkylrest steht,

B für einen Pyrrolyl-, einen substituierten Phenylrest der Formel II oder einen substituierten Pyridinrest der Formel III

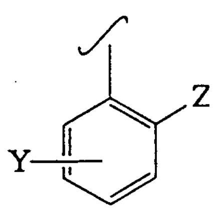

(II)

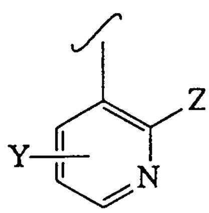

(III)

worin Z eine Hydroxyl-, Amino- oder eine Mercaptogruppe und Y ein Wasserstoffatom, einen Carboxy- oder einen Sulfonylrest bedeuten

oder für einen Nitrosomethylrest der Formel IV

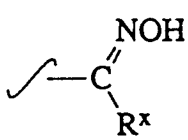

(IV)

steht,

worin $R^x$ einen $C_{1-6}$-Alkylrest, der gegebenenfalls zusammen mit $R^4$ über eine Trimethylen- oder Tetramethylengruppe zu einem 5- bzw. 6-Ring cyclisiert ist, bedeutet

und A für einen Amino-, einen Mercapto-, einen Carboxy-, einen $C_{2-6}$-Alkinyl- oder -Alkenyl-, einen

Oxiranyl-, einen fluorierten Phenoxycarbonyl-, einen gegebenenfalls mit einem Natriumsulfatrest substituierten Succinimidoxycarbonyl-, einen Aminophenyl- oder Isothiocyanatophenylrest steht,

wobei die Phenylgruppe gegebenfalls zusätzlich durch einen Carboxy-, einen Chlorsulfonyl- oder einen Sulfonsäurerest substituiert sein kann

oder eine - mit Hilfe der genannten funktionellen Gruppe gegebenenfalls über einen bifunktionellen Linkerrest L gebundene - sich selektiv in Läsionen oder bestimmten Geweben anreichernde Verbindung T enthält,

wobei T für monoklonale Antikörper oder deren Fragmente, Hormone, Enzyme, Liganden für Zellmembranrezeptoren, Neurotransmitter, Lipide, Steroide, Saccharide, Aminosäuren und Oligopeptide, Biotin, sowie Radiosensitizer, wie z.B. Misonidazol steht,

wobei im Falle von

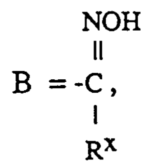

Pyrrolyl- oder Y = H, $R^2$ für eine hydroxilierte $C_{1-6}$-Alkylenkette oder A für einen substituierten Aminophenyl- oder Isothiocyanatophenylrest stehen sollen

sowie deren Komplexe mit- zur Diagnostik und Tumortherapie geeigneten - radioaktiven Metallionen, sowie deren Salze mit anorganischen und organischen Säuren,

**dadurch gekennzeichnet**, daß man
a) ein 1,3-Propandiamin der allgemeinen Formel V

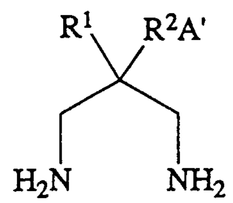
(V)

worin $R^1$ und $R^2$ die oben genannten Bedeutungen haben, und A' A oder ein in A umwandelbarer Rest bedeutet
mit einer Verbindung der allgemeinen Formel VI

$B - R^4C = O$ (VI)

in der $R^4$ und B die oben genannten Bedeutungen haben,

in einem polaren Lösungsmittel, vorzugsweise Ethanol, oder unter Verwendung eines Wasserabscheiders in einem unpolaren Lösungsmittel, vorzugsweise Benzol, bei Temperaturen von 25 - 18°C innerhalb von 6 Stunden bis 3 Tagen umsetzt,

die Iminofunktion in an sich bekannter Weise, vorzugsweise mit Natriumborhydrid in einem polaren Lösungsmittel, vorzugsweise einem Methanol/Wasser-Gemisch, bei Temperaturen von 25 - 100°C innerhalb von 0,5 bis 24 Stunden, vorzugsweise 2 Stunden, reduziert,

oder

b) indem man ein Propandiamin der allgemeinen Formel V

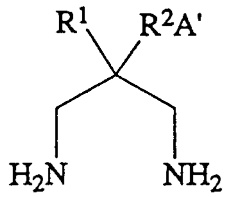
(V)

worin $R^1$, $R^2$ und A' die oben genannten Bedeutungen haben, mit einer Verbindung der allgemeinen Formel VII

B - CO-X (VII),

in der B die oben genannte Bedeutung hat, in B enthaltene funktionelle Gruppen gegebenenfalls in geschützter Form vorliegen und X für ein Halogenatom, vorzugsweise für ein Chloratom steht,

oder

c) indem man ein Propandiamin der allgemeinen Formel V

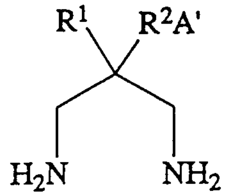
(V)

worin $R^1$, $R^2$ und A' die oben genannten Bedeutungen haben, mit einer Verbindung der allgemeinen Formel VIII

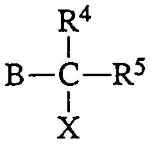
(VIII)

in der $R^4$, $R^5$, B und X die oben genannten Bedeutungen haben und in B enthaltene funktionelle Gruppen gegebenenfalls in geschützter Form vorliegen,

oder

d) substituierte Malonsäurehalogenide, vorzugsweise Malonsäurechloride der allgemeinen Formel IX,

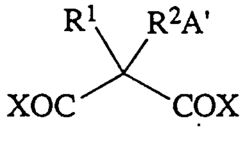
(IX)

worin $R^1$, $R^2$, A' und X die oben genannten Bedeutungen haben und in B enthaltene funktionelle Gruppen gegebenenfalls in geschützter Form vorliegen, mit einem Amin der allgemeinen Formel X

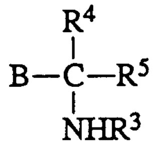

(X)

in denen $R^4$, $R^5$ und B die oben genannten Bedeutungen haben, $R^3$ für ein Wasserstoffatom oder für einen $C_{1-6}$-Alkylrest steht und in B enthaltene funktionelle Gruppen gegebenenfalls in geschützter Form vorliegen,

in einem aprotischen Lösungsmittel, vorzugsweise Dichlormethan, bei Temperaturen von 0 - 180 °C, vorzugsweise bei Raumtemperatur, innerhalb von 2 bis 24 Stunden, vorzugsweise 4 Stunden, gegebenenfalls unter Zusatz von geeigneten Basen, z.B. Triethylamin, umsetzt,

die nach Verfahren d) erhaltene Amidfunktion in an sich bekannter Weise, vorzugsweise mit Boran in THF oder mit Lithiumaluminiumhydrid in einem aprotischen Lösungsmittel, vorzugsweise Diethylether, bei Temperaturen von 25 - 15 °C innerhalb von 0,5 bis 24 Stunden, vorzugsweise 8 Stunden, zur entsprechenden Aminofunktion reduziert,

die Aminogruppen, im Falle daß $R^3$ für ein Wasserstoffatom steht, gegebenenfalls in an sich bekannter Weise mit einem Alkylierungsmittel alkyliert und vorhandene Schutzgruppen abspaltet

und in den so erhaltenen Verbindungen die Gruppe A' gegebenenfalls zu A umwandelt- gegebenenfalls nach Schutz der freien Aminogruppen und der funktionellen Gruppen Z mit Schutzionen, z.B. als Cu-Komplex - generiert und anschließend gewünschtenfalls die so erhaltenen Verbindungen über diese funktionelle Gruppe an sich selektiv anreichernde Verbindungen T koppelt und den Substituenten B gewünschtenfalls mit dem jeweils gewünschten radioaktiven Isotop komplexiert, wobei vorher im Produkt gegebenenfalls vorhandene Schutzionen nach an sich literaturbekannten Methoden entfernt werden und die Reihenfolge der Schritte Komplexierung mit radioaktiven Isotopen und Kopplung an T vertauscht werden kann.

Europäisches Patentamt

# EUROPÄISCHER RECHERCHENBERICHT

Nummer der Anmeldung

EP 92 25 0051

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,P, X | EP-A-0 417 870 (INSTITUT FÜR DIAGNOSTIKFORSCHUNG GMBH)<br>* Ansprüche 1,3-7 * | 1,3-7 | C07C215/50<br>C07C251/38<br>C07C309/46<br>C07C323/32<br>C07C331/28<br>C07D207/40<br>C07D213/70<br>A61K49/02<br>A61K43/00 |
| A | EP-A-0 369 497 (SALUTAR, INC.)<br>* Seite 3, Zeile 40 - Seite 4, Zeile 22 * | 1 | |
| A | EP-A-0 292 761 (SALUTAR, INC.)<br>* Ansprüche 1,10,13,14 * | 1,6 | |
| A | EP-A-0 290 047 (SALUTAR, INC.)<br>* Ansprüche 1,6,13 * | 1,6 | |
| A,D | EP-A-0 194 843 (AMERSHAM INTERNATIONAL PLC)<br>* Anspruch 1 * | 1 | |
| A | EP-A-0 123 504 (AMERSHAM INTERNATIONAL PLC)<br>* Ansprüche 1,3-5 * | 1 | |
| A | WO-A-8 600 898 (UNIVERSITY OF FLORIDA)<br>* Seite 89, Verbindungen 211, 212, 213 * | 1 | RECHERCHIERTE SACHGEBIETE (Int. Cl.5) |
| A,D | EP-A-0 200 492 (PRESIDENT AND FELLOWS OF HARVARD COLLEGE)<br>* Ansprüche 1,25 * | 1,6 | A61K<br>C07C<br>C07D |

Der vorliegende Recherchenbericht wurde für alle Patentansprüche erstellt

| Recherchenort | Abschlußdatum der Recherche | Prüfer |
|---|---|---|
| BERLIN | 17 JUNI 1992 | Christian Hass |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument